# EUROPEAN PATENT APPLICATION

(11) **EP 4 534 649 A1**
(43) Date of publication of application: **09.04.2025**
(21) Application number: 23201384.7
(22) Date of filing: 03.10.2023
(51) Int. Cl.: C12N 5/071, G01N 33/50

(54) **A METHOD OF QUALIFYING AS PHARMACEUTICALLY ACTIVE A BATCH OF UNDIFFERENTIATED ADULT STEM CELLS HAVING THE POTENTIAL TO DIFFERENTIATE ALONG THE HEPATIC LINEAGE**

(71) Applicant: Unicyte AG, 6370 Oberdorf (CH)
(72) Inventor: HERRERA-SANCHEZ, Maria Beatriz, 61352 BAD HOMBURG (DE); SACCU, Gabriele, 61352 BAD HOMBURG (DE); TAPPARO, Marta, 61352 BAD HOMBURG (DE)
(74) Representative: Comoglio, Elena

(57) **Abstract**

The invention relates to a qualification method to qualify a batch of undifferentiated adult stem cells having the potential of differentiating along the hepatic lineage as pharmaceutically active, wherein pharmaceutically active indicates that the batch is actually capable of producing sufficiently differentiated and/or functional and/or viable hepatocyte-like cells, so that the undifferentiated adult stem cells in said batch or the extracellular vesicles (EVs) derived therefrom are suitable for use in the therapeutic treatment of a subject suffering from a health condition or disease or are suitable to establish a Master Cell Bank from which sufficiently differentiated and/or functional and/or viable hepatocyte-like cells may subsequently be produced.

## Description

The present invention relates to a method of qualifying as pharmaceutically active a batch of undifferentiated adult stem cells having the potential to differentiate along the hepatic lineage, such as a batch of human liver pluripotent progenitor cells or bone marrow mesenchymal stem cells.

### Background of the invention

Liver transplantation is considered to be the main reliable treatment for patients suffering from end-stage liver disease, nevertheless, the shortage of available organs, high cost, risk of rejection, and requirement of immunosuppression has increased the interest to develop alternative approaches such as cell therapy. The primary source of cells that have been investigated were hepatocytes, however there are several limitations in the *in vivo* use of hepatocytes, namely low *in vitro* expansion ability, rapid loss of function when cultured *in vitro,* and substantial loss of vitality after thawing from cryo-storage. Moreover, there is restriction to obtain sufficient amounts of hepatocytes for human treatment due to limited access to human liver tissues.

Recently, a few research groups have studied the therapeutic potential of stem cells as an alternative source to obtain mature hepatocytes. Among the main advantages, stem cells are easy to culture and expand *in vitro,* maintain their plasticity after expansion, possess limited karyotype modification, and are able to differentiate into hepatocyte-like cells under specific *in vitro* culture conditions (Herrera MB, Fonsato V, Bruno S, et al. Human liver stem cells improve liver injury in a model of fulminant liver failure. Hepatology. 2013;57(1):311-319. doi:10.1002/hep.25986).

Different sources of stem cells have been considered; however, they have various advantages and disadvantages. For example, embryonic stem cells (ESCs) are a promising source of cells and many groups have demonstrated the functionality of ESC hepatocyte-like cells *in vivo.* However, their use remains restricted in many countries due to legal issues and ethical concerns. An alternative to ESCs are the Induced pluripotent stem cells (iPSCs), but their disadvantages include high cost and safety concerns that constrain use for further clinical applications.

Human liver stem cells (HLSCs) are liver progenitor cells that possess high *in vitro* self-renewing capabilities (Herrera MB, Bruno S, Buttiglieri S, et al. Isolation and characterization of a stem cell population from adult human liver. Stem Cells. 2006;24(12):2840-2850. doi: 10. 1634/stemcells. 2006-0 114). They are hepatic committed cells, that express human albumin, alpha-fetoprotein, and variable expression of CK8 and CK18. These cells also express markers of multidirectional differentiation such as Nanog, Oct3/4, Sox2 and Musashi (Herrera MB, Fonsato V, Bruno S, et al. Human liver stem cells improve liver injury in a model of fulminant liver failure. Hepatology. 2013;57(1):311-319. doi:10.1002/hep.25986). Similarly to mesenchymal stem cells, HLSCs are positive for CD90, CD105, CD29, CD73 and lack CD34, CD14 and CD45. Besides preclinical *in vivo* studies, the clinical safety of these cells was investigated recently in the first human Phase I clinical trial in infants with inherited neonatal-onset hyperammonemia (Spada M, Porta F, Righi D, et al. Intrahepatic Administration of Human Liver Stem Cells in Infants with Inherited Neonatal-Onset Hyperammonemia: A Phase I Study. Stem Cell Rev Rep. 2020;16(1):186-197. doi:10.1007/s12015-019-09925-z). HLSCs were administered percutaneously into the liver parenchyma under ultrasound guidance and local anesthesia. This minimally invasive approach was possible because of the small injection-volumes providing the individual HLSC doses locally. In these patients treated with intrahepatic HLSCs, no immunosuppressive regimen was planned, and no local or systemic adverse events were registered. This was based on the evidence of not only the low immunogenicity of HLSCs, but also on their immunomodulatory abilities that may potentially obviate rejection (Bruno S, Grange C, Tapparo M, et al. Human Liver Stem Cells Suppress T-Cell Proliferation, NK Activity, and Dendritic Cell Differentiation. Stem Cells Int. 2016;2016:8468549. doi:10.1155/2016/8468549). Indeed, HLSCs do not express HLA class II or costimulatory molecules such as CD40, CD80, and CD86. Moreover, HLSCs inhibit T cell proliferation and dendritic cell differentiation similar to mesenchymal stem cells. In addition, HLSCs were found to suppress natural killer degranulation protecting them from allogeneic lysis.

### Description of the invention

A shortage of functional hepatocytes may hamper therapeutic applications, as mature hepatocytes cannot be expanded and tend to lose functionality *in vitro.* On the other hand, as discussed above, human liver pluripotent progenitor cells, such as HLSCs (Human Liver Stem Cells), or bone marrow mesenchymal stem cells are known to be able to differentiate into hepatocyte-like cells under cell culture conditions in the presence of various combinations of cytokines and other molecules known to play a role in hepatocyte maturation. However, it is also known that cells and tissues originating from different donors may show considerable differences in terms of viability and functionality. This also applies to hepatocyte-like cells derived from stem cells from different donors, as well as to different batches of hepatocyte-like cells derived from the same donor.

Accordingly, a qualification method would be needed to determine whether a batch of starting material, namely undifferentiated adult stem cells having the potential to differentiate along the hepatic lineage is actually capable of differentiating giving rise to functional hepatocyte-like cells. In fact, only in this case the batch would be worth pursuing in the pharmaceutical development process, otherwise it should be discarded.

In order to meet this and other needs, the present invention provides a method of qualifying as pharmaceutically active a batch of undifferentiated adult stem cells that have the potential of differentiating along the hepatic lineage as defined in appended claim 1. Further features and advantages of the qualification method of the invention are defined in the dependent claims. All of the appended claims for an integral part of the description.

Within the present description, the expressions "undifferentiated adult stem cells" indicate a group of not differentiated or not fully differentiated stem cells that possess the abilities of self-renewal, multipotent differentiation, and repair after organ injury. They are labeled as "adult" because they are isolated from an adult tissue, such as liver or bone marrow, in contrast with "embryonic stem cells" which are isolated from the inner cell mass of a blastocyst. Adult stem cells are also known as "somatic stem cells".

Within the present description, the expression "qualification as pharmaceutically active" or "qualified as pharmaceutically active" means that the batch of undifferentiated adult stem cells having the potential to differentiate along the hepatic lineage has been qualified as being actually capable of producing sufficiently differentiated and/or functional and/or viable hepatocyte-like cells, so that the undifferentiated adult stem cells in said batch or the extracellular vesicles (EVs) derived therefrom are suitable for use in the therapeutic treatment of a subject suffering from a health condition or disease.

In therapeutic applications, the batch of undifferentiated adult progenitor cells qualified as pharmaceutically active or the EVs derived therefrom may either be directly administered to the subject suffering from the health condition or disease, or the batch may be used to establish a Master Cell Bank, from which progenitor/stem cell cultures or EVs suitable for use in therapy may be subsequently produced.

As is known, mammalian cells, including human cells, are quite vulnerable to genetic variation and contamination if indefinitely maintained in culture by serial passages. Fortunately, the mammalian cells can be cryopreserved and stored in a stable condition at ultra-low temperatures until required. Thus, a Master Cell Bank is defined as an aliquot of cells prepared from the original biological material, such a selected cell clone, or a tissue or a tumor from a donor, which is dispensed into one or multiple containers and stored under defined conditions, and from which progenitor/stem cell cultures may be subsequently obtained.

When the qualification method according to the invention is used to determine whether a given batch of undifferentiated adult stem cells is pharmaceutically active in the sense that it is suitable to establish a Master Cell Bank for subsequent production of pharmaceutically active materials such as cells or EVs, testing may be performed immediately after harvest or may be performed upon formulation, freezing and thawing. If the tested batch passes the test and is qualified as pharmaceutically active, it may then be used to establish a Master Cell Bank, otherwise it shall be discarded. Of course, during the pharmaceutical development process further qualification may be performed at any given stage without limitation.

The undifferentiated adult stem cell batch that may be tested with the qualification method of the invention may be, without limitation, a human liver pluripotent progenitor cell batch or a bone marrow mesenchymal stem cell batch. The ability of human liver pluripotent progenitors to differentiate along the hepatic lineage is known from the prior art. WO2006126219 discloses human non-oval liver stem cells which are characterized by multilineage differentiation abilities, including the ability to differentiate along the hepatic lineage, as well as their isolation from adult liver tissue. WO2006126219 also discloses the cell marker expression profile of a specific cell line of the aforementioned non-oval liver stem cells, which is named as "HLSC". The marker expression profile of the HLSC cell line is shown in Table I of WO2006126219, which is herein incorporated by reference. The differentiation of bone marrow mesenchymal stem cells is disclosed in the prior art too.

According to the invention, in step i) of the qualification method a sample of the undifferentiated adult stem cell batch to be tested is obtained and cultured in a hepatic differentiation medium, whereby hepatocyte-like cells are obtained. The specific culture medium which is employed at this step of the qualification method is not limiting, as long as it is a culture medium containing a combination of growth factors and/or other molecules capable of bringing about differentiation along the hepatic lineage. Hepatic differentiation media are disclosed both in the experimental part of the present patent application and in the prior art. For example, Najimi M, Khuu DN, Lysy PA, et al. Adult-Derived Human Liver Mesenchymal-Like Cells as a Potential Progenitor Reservoir of Hepatocytes? Cell Transplantation. 2007;16(7):717-728. doi:10.3727/000000007783465154 discloses a serum-containing hepatic differentiation medium including epidermal growth factor, insulin and dexamethasone; Herrera MB, Fonsato V, Bruno S, et al. Human liver stem cells improve liver injury in a model of fulminant liver failure. Hepatology. 2013;57(1):311-319. doi:10.1002/hep.25986 discloses a serum-containing hepatic differentiation medium including hepatocyte growth factor and fibroblast growth factor 4.

According to the invention, in step ii) of the qualification method the hepatocyte-like cells obtained in step i) are subjected to a functional assay comprising one or a combination of the following assays:
- detection of Indocyanine green (ICG) uptake,
- detecting the expression of the transporter OATP1B1, and/or
- detection of the expression of one or more markers selected from the group consisting of Nanog, SOX2, KLF4, OCT3/4, CK8, αFP, Cyp1A1, CYP3A4, Cyp7A1, FVIII, FIX, FXI, ASL and ARG,
to which detection of urea secretion may be optionally added.

In step iii) of the qualification method of the invention, the same one or more functional assays are performed on a negative control cell sample. According to an embodiment, a negative control cell sample is a sample of undifferentiated adult stem cells having the potential of differentiating along the hepatic lineage, but which are not cultured in a hepatic differentiation medium as defined above. Preferably, such undifferentiated adult stem cells which are not cultured in a hepatic differentiation medium are the same undifferentiated adult stem cells that are cultured in a hepatic differentiation medium and tested. According to another embodiment, a negative control cell sample is a sample of cells that do not have the potential of differentiating along the hepatic lineage. Preferably, such cells are cultured under hepatic differentiation conditions, but this does not result in differentiation into hepatocyte-like cells. As a non-limitative example, cells that do not have the potential of differentiating along the hepatic lineage are HaCaT keratinocytes.

The assays identified in the appended claims are based on different mechanisms of action to show functionality (except the ICG assay and the OATP1B1 assay which are both used to evaluate liver functionality). While each single one of the assays mentioned in appended claim 1 is sufficient to qualify the undifferentiated adult stem cells, the combination of two or more of them can establish the functionality of the undifferentiated adult stem cells based on more than one mechanism of action and therefore enhance the confidence level in the qualification assay of the invention.

Within the present description, the expression "not having the potential of differentiation along the hepatic lineage" indicate cells that are not capable of differentiating along the hepatic lineage and give rise to hepatocyte-like cells even when placed under hepatic differentiation conditions, e.g., cultivation in a hepatic differentiation medium as disclosed herein.

In step iv) of the qualification method of the invention, the results of the functional assay obtained in steps ii) and iii) are compared to each other. That is to say, the results obtained with the undifferentiated stem cell batch to be qualified are compared with the results obtained with the negative control cell sample. If such results are statistically significantly different, the tested batch is qualified as being pharmaceutically active. Statistical significance is assessed using a p-value of ≤ 0.05.

The qualification method of the invention may further include testing a positive control sample, which is common practice in this type of assays and needs no further description. As explained above, in the present description "pharmaceutically active" either means that the undifferentiated adult stem cell batch is capable of giving rise to sufficiently functional and/or differentiated and/or viable hepatocyte-like cells which are therefore therapeutically effective, or that the said batch is suitable for use in establishing a Master Cell Bank from which to therapeutically effective hepatocyte-like cells or EVs may be subsequently obtained.

Within the present description, the expression "therapeutically effective" means suitable for treating a health condition in a human subject in need thereof, wherein the health condition is preferably a fibrotic, a metabolic, or an oncologic disease, more preferably a fibrotic, a metabolic or an oncologic disease of any tissue type the undifferentiated adult stem cells are capable of differentiating into. Non limiting examples are liver and liver-associated tissues (wherein liver and liver associated tissues include e.g. liver parenchymal cells such as hepatocytes and bile duct cells and/or non-parenchymal liver cells such as sinusoidal endothelial cells, Kupffer cells and/or hepatic stellate cells), the biliary tree, pulmonary tissues, kidney tissues, heart tissues, pancreatic tissues. More preferably, the health condition is selected from the group consisting of urea cycle disorders, Crigler-Najiar, alphalantitrypsin deficiency, biliary atresia, acute liver failure, nonalcoholic fatty liver disease, nonalcoholic steatohepatitis, primary biliary cholangitis, autoimmune hepatitis, cirrhosis, hepatocellular carcinoma, cholangiocarcinoma, liver genetic metabolic disorders, primary sclerosing cholangitis, chronic kidney disease, focal segmental sclerosis, diabetic nephropathy, membranous glomerulonephritis, diabetes type I and II, cardiac fibrosis, idiopathic pulmonary fibrosis, metastatic hepatic tumors, renal cell carcinoma, lung carcinoma.

### Drawings description

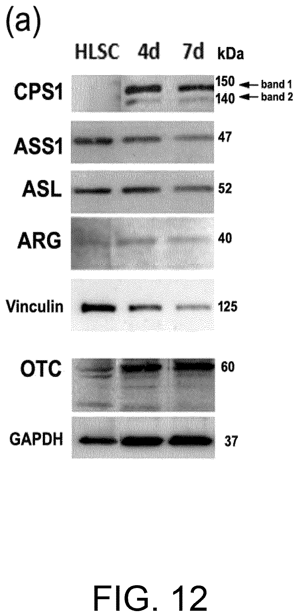

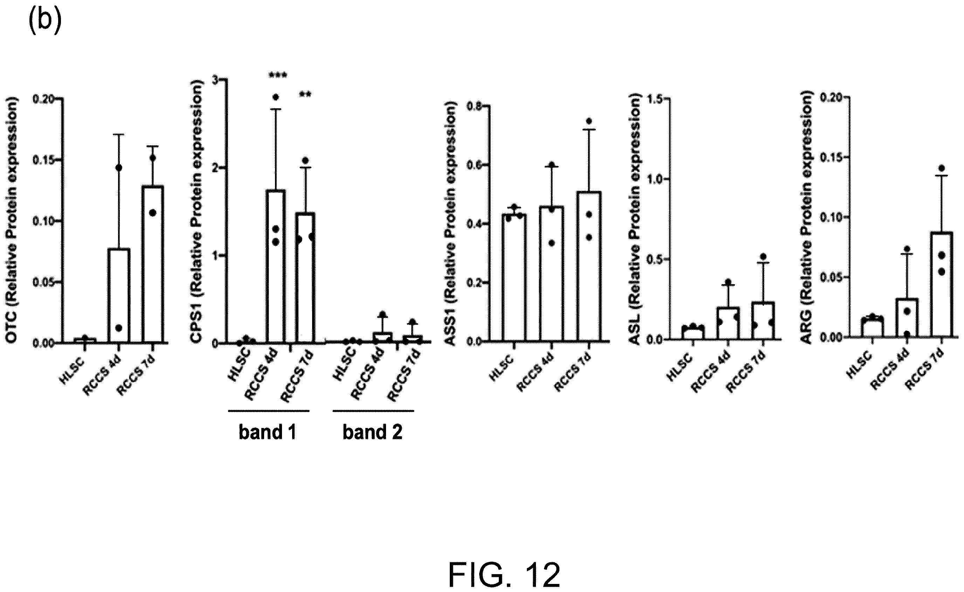

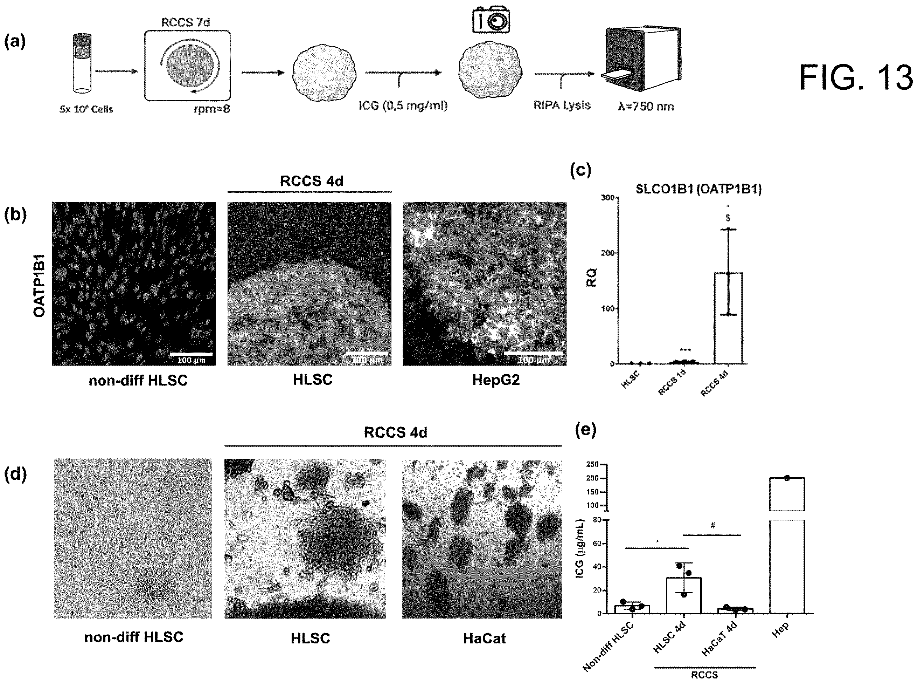

The invention is described in more details in the following non limiting experimental section, which is provided for illustration only and refers to the following figures:

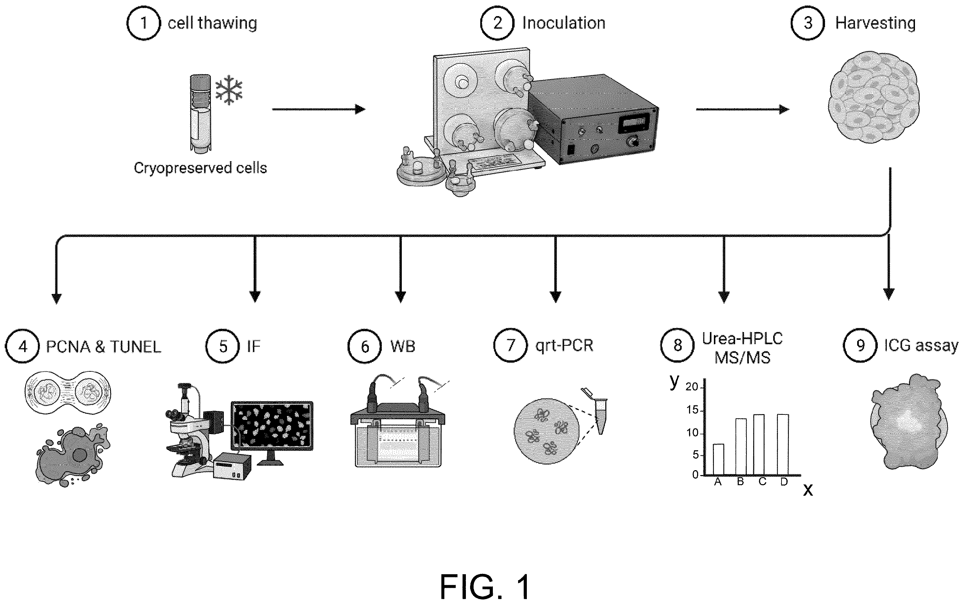

Figure 1. Overview of the experiment

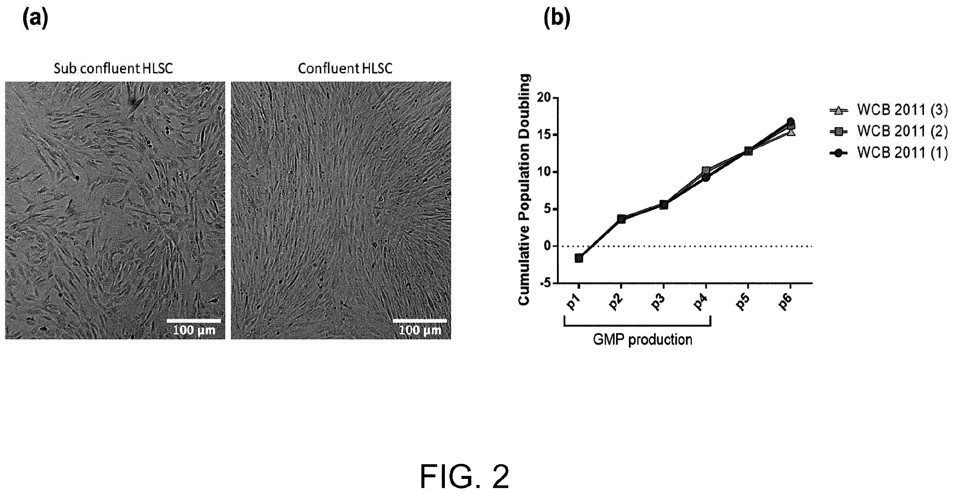

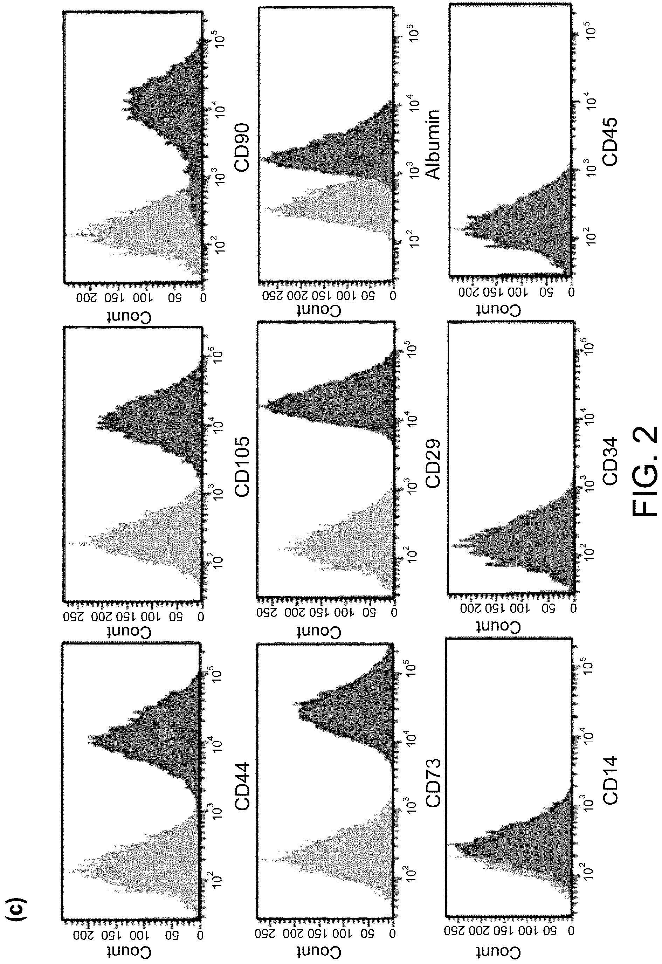

Figure 2. HLSC characterization, a) Representative micrograph showing HLSC morphology. b) Cumulative population doubling of 3 different cell batches of HLSC from passage 0 to 7. c) Representative FACS analysis for the positive surface markers CD44, CD105, CD73, CD90, CD29 and Albumin and the negative markers CD34, CD45, and CD14

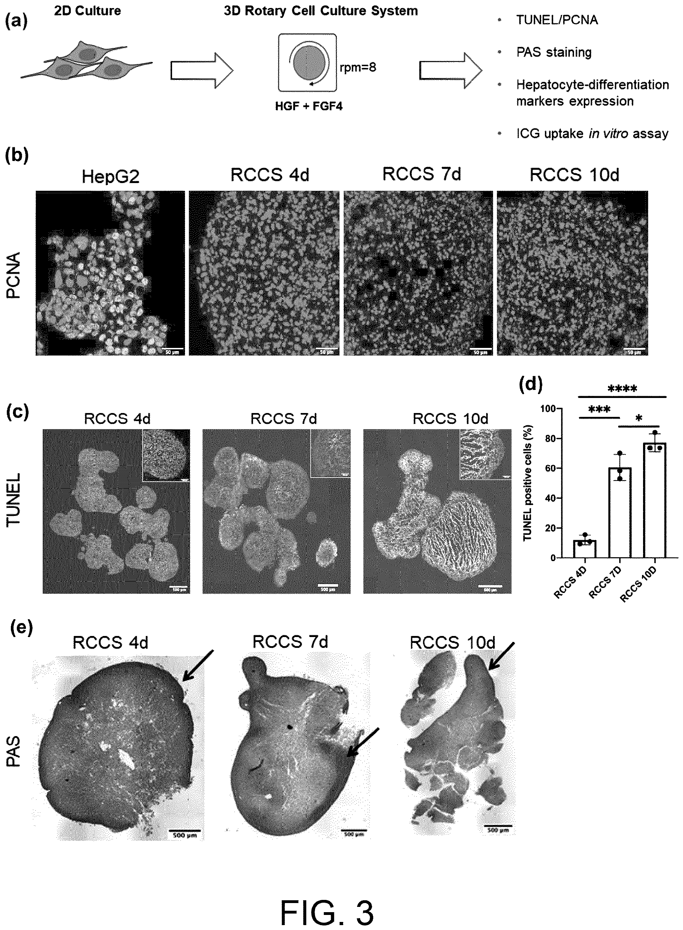

Figure 3. *In vitro* differentiation of HLSC into hepatocyte-like cells in RCCS. a) Experimental layout. b) Representative PCNA staining at day 4, 7 and 10 of differentiation of HLSC in RCCS. Scale bar 50 µm. HepG2 cell line was used as positive control. c) Apoptotic cell death was detected by TUNEL (green), and the nucleus (blue) was stained with DAPI. Scale bars = 500 µm. d) Quantification of TUNEL-positive cells in all the experimental groups. Values represent the mean ± SD from 3 independent experiments (black dots represent the experimental replicates). RCCS 4d vs. RCCS 7d *** p<0.0001, RCCS 7dvs. RCCS 10d *p < 0.05, RCCS 4d vs. RCCS 10d ****p < 0.001, (one-way analysis of variance). e) PAS staining of RCCS aggregates analyzed at day 4, 7 and 10 of differentiation. Scale bar 500 µm

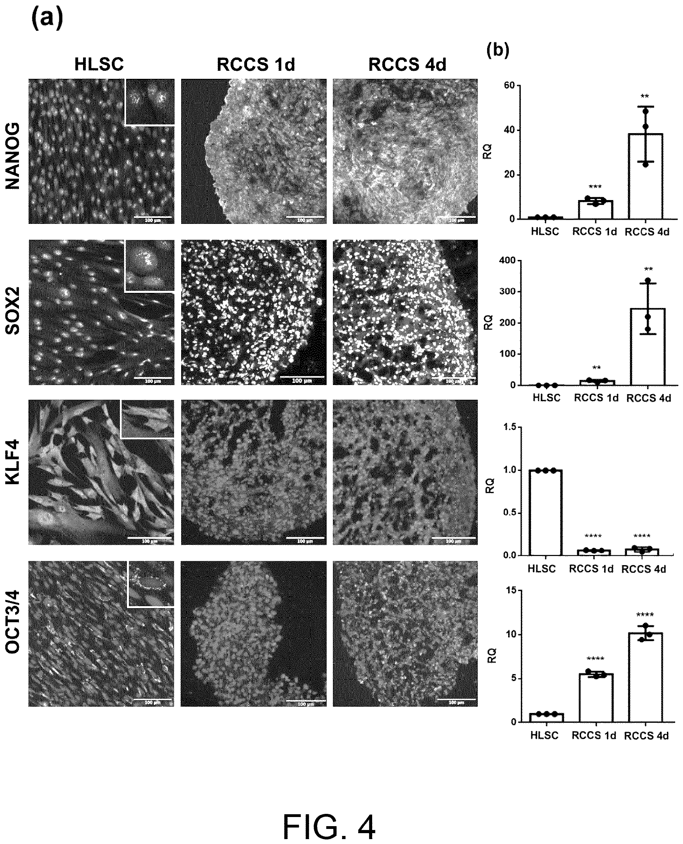

Figure 4. Stem cell markers. a) Representative IF micrograph of stem cell markers Nanog, Sox2, KLF4, OCT4 in undifferentiated HLSC and RCCS differentiated HLSC. Scale bar 100µm, magnification 40X. Nuclei are stained in blue with DAPI. b) Real-time PCR of the embryonic stem cells genes. Results for the differentiated HLSC were normalized using TBP and to undifferentiated HLSC (experimental negative control). Results are expressed as Relative Quantification (RQ). The results are presented as mean values of 3 experiments ±SD. ** p<0.01, *** p<0.001, **** p<0.0001 RCCS 1d and 4d vs. HLSC

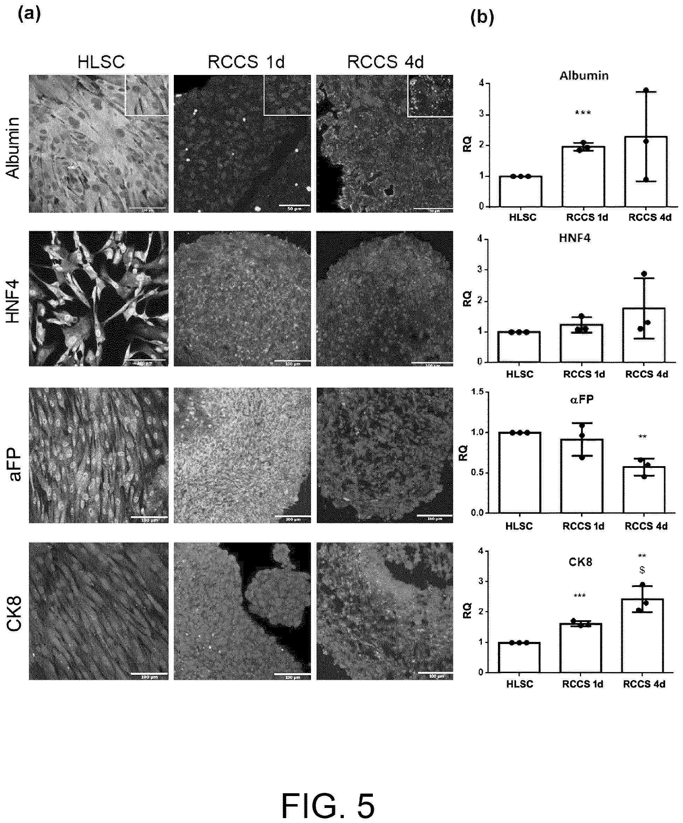

Figure 5. Hepatic markers. a) Representative immunofluorescence micrograph of Albumin, HNF4, αFP and CK8 in HLSC and HLSC aggregates after differentiation in RCCS. Scale bar 100µm, magnification 40X. Nuclei were stained in blue with DAPI. b) Real-time PCR array data analysis of hepatic markers of undifferentiated and differentiated HLSC. Results are expressed as Relative Quantification (RQ), data were normalized using TBP and to undifferentiated HLSC (experimental negative control). The results are presented as mean values of 3 experiments ±SD *** p<0.001 vs HLSC

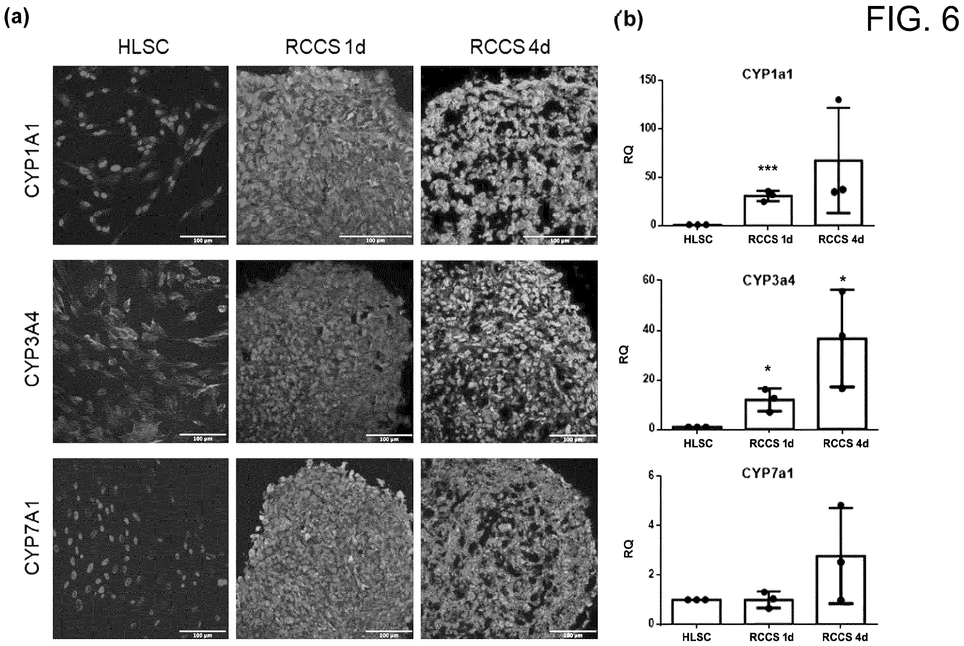

Figure 6. Hepatic markers. a) Representative immunofluorescence micrograph data of cytochrome P450 isoform CYP1a1, CYP3a4, and CYP7a1 in HLSC and HLSC aggregates after differentiation in RCCS. Scale bar 100µm. Magnification 40X. Nuclei are stained in blue with DAPI. b) Real-time PCR of cytochromes P450 isoenzymes genes. Results for the differentiated HLSC were normalized using TBP and to undifferentiated HLSC (experimental negative control). Real time data are expressed as Relative Quantification (RQ). The results are presented as mean values of 3 experiments ±SD. * p<0.05, ***p<0.001 vs. HLSC

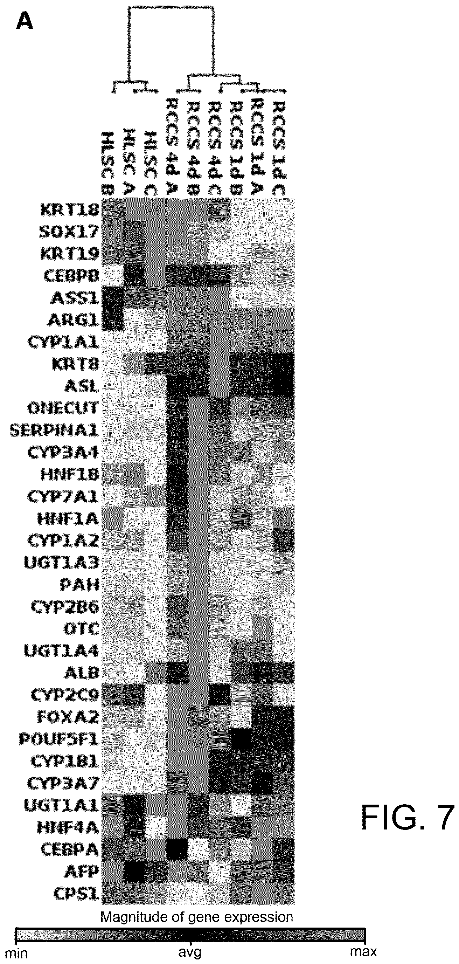

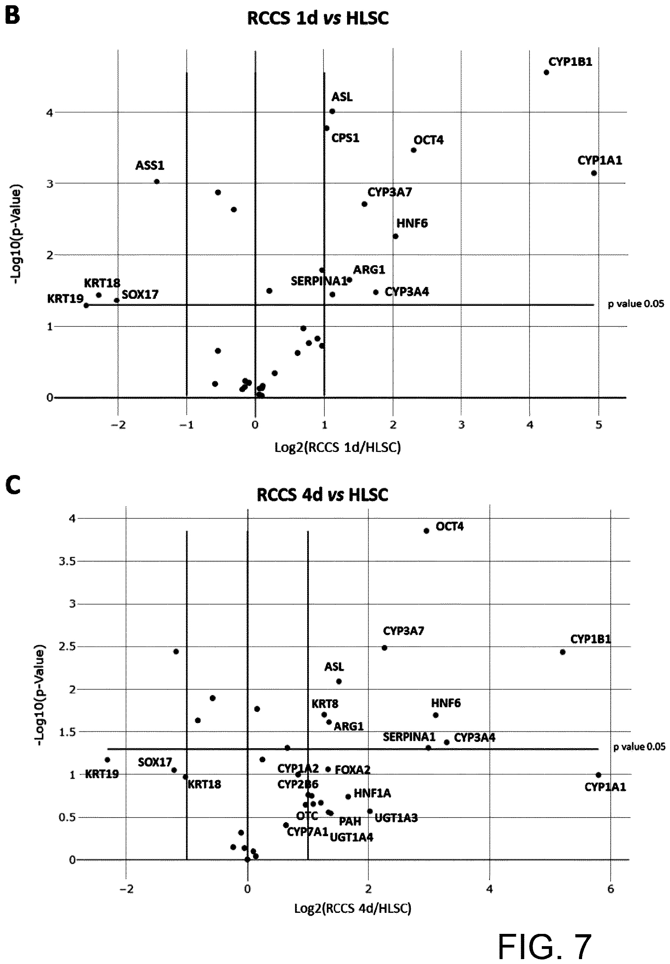

Figure 7. A) Heatmap of gene expression showed that the three groups analyzed; RCCS 1d and RCCS 4d cluster together and HLSC are separated from RCCS samples. B) Volcano plot comparing gene expression of HLSC vs RCCS day1 and RCCs day4. Red dots represent up-regulated genes and green dots represent downregulated genes. All dots above the p-value line are statistically significant in respect to control

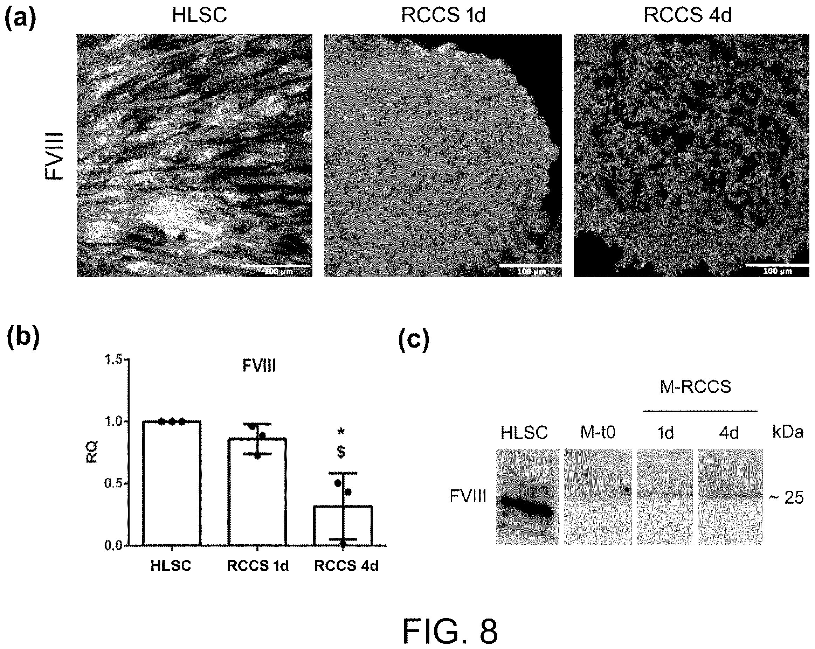

Figure 8. Expression of Hepatic markers. a) Representative immunofluorescence micrograph data of coagulation factor FVIII in HLSC and HLSC aggregates after differentiation in RCCS. Scale bar 100µ. Nuclei are stained in blue with DAPI. b) Real-time PCR of coagulation factors gene. (c) Representative Western Blot of FVIII in HLSC and HLSC supernatants after differentiation. M-t0: differentiation media at t0 (without HLSC), supernatants after 1 and 4 days of cell differentiation (M-RCCS). Results for the differentiated HLSC were normalized using TBP and to undifferentiated HLSC (experimental negative control). Real time data are expressed as Relative Quantification (RQ). The results are presented as mean values of 3 experiments ±SD. * p<0.05 RCCS 4d vs. HLSC

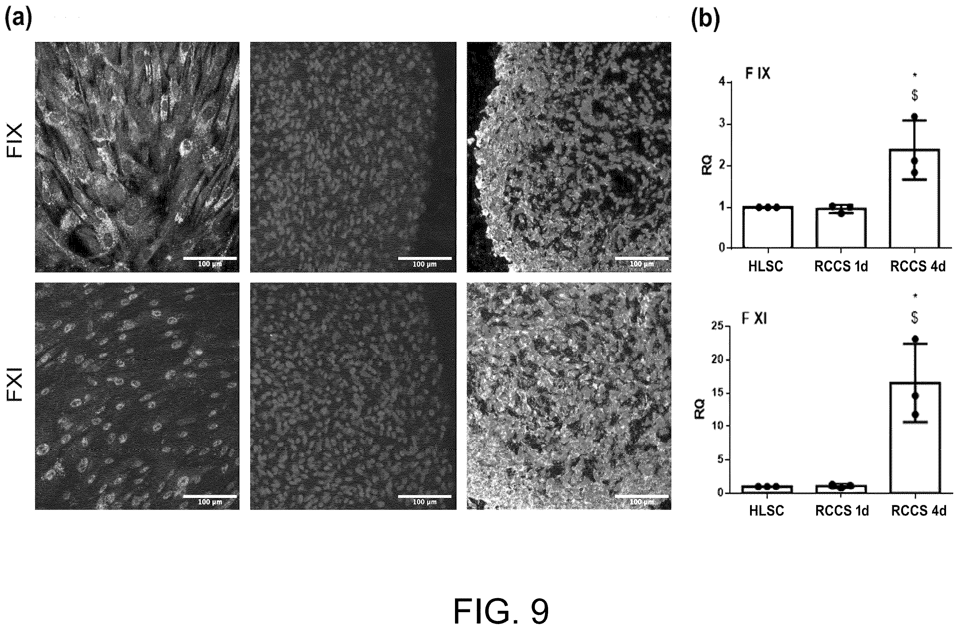

Figure 9. Coagulation Factor expression. Representative immunofluorescence micrograph data of coagulation factor XI (FXI) and FIX in HLSC and HLSC aggregates after differentiation in RCCS. Scale bar 100µm. Nuclei are stained in blue with DAPI. b) Real-time PCR of coagulation factor genes. Results for the differentiated HLSC were normalized using TBP and to undifferentiated HLSC (experimental negative control). Real time data are expressed as Relative Quantification (RQ). The results are presented as mean values of 3 experiments ±SD. * p<0.05 RCCS 4d vs. HLSC

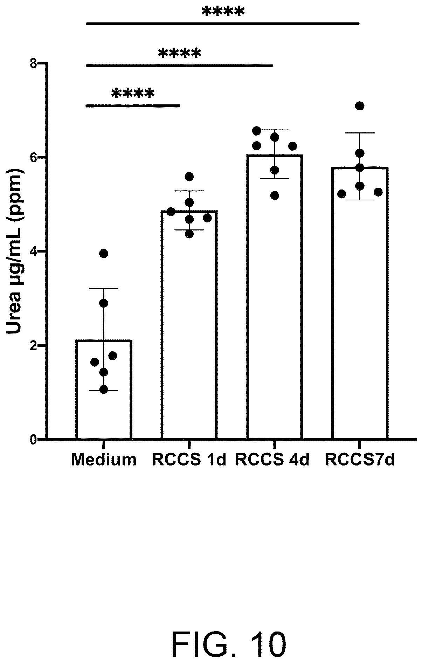

Figure 10. Increase in hepatocyte function of HLSC under different culture conditions. The amount of Urea secretion was quantified by HPLC MS/MS analysis. Results were expressed as ppm (part per million) (µg/mL) of Urea. The results are presented as mean values of 6 independent experiments ± SD. **** p<0.0001 vs. HLSC

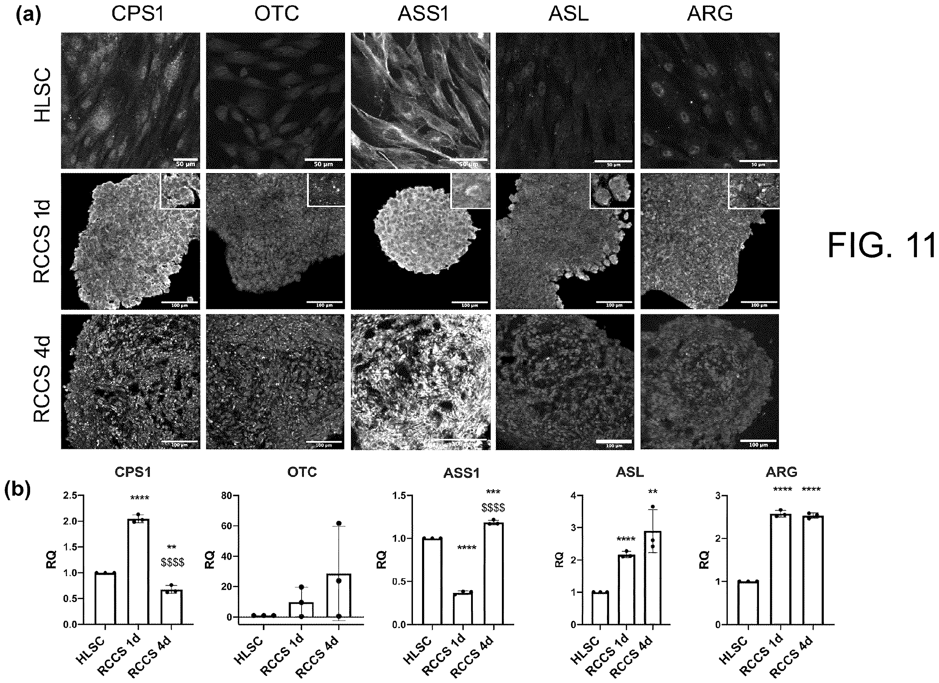

Figure 11. Expression of Urea cycle enzymes. a) Representative immunofluorescence micrographs of the urea cycle enzymes CPS, OTC, ASS1, ASL and ARG1 in HLSC and differentiated HLSC in RCCS. Scale bar 50 and 100 µm. Nuclei are stained in blue with DAPI. b) Real-time PCR data of urea cycle enzymes genes in HLSC and differentiated HLSC. Data are expressed as Relative Quantification (RQ) of 3 independent experiments ±SD. ** p<0.01, ***, p<0.001, **** p<0.0001 RCCS 1d and RCCS 4d vs. HLSC, $$$$ p<0.0001 RCCS 4d vs. RCCS 1d
Figure 12. Urea cycle proteins levels. Western blot analysis of Urea cycle enzymes. a) Representative blot. b) Relative amounts of proteins counted in relation to HLSC. The results are presented as mean values of 3 independent experiments and are normalized using Vinculin and GAPDH as housekeeping proteins ± SD. *** p<0.001 vs. HLSC
Figure 13. Indocyanine green update (ICG) assay. a) Schematic representation of the assay b) Representative IF micrograph of OATP1B1 in HLSC and RCCS differentiated cells at day 4. HepG2 cells were used as control. Nuclei are stained in blue with DAPI. 40X magnification. c) Real time PCR data of OATP1B1 (SLCO1B1), result is expressed as Relative Quantification ±SD. * p<0.05, *** p≤0.001 vs. HLSC; $ p<0.05 vs. HLSC 1d. (d) Representative micrographs of non-differentiated, differentiated HLSC and HaCat showing uptake of ICG 1 hour after ICG treatment. 4X magnification. e) ICG quantification in non-differentiated HLSC, HLSC and HaCat after 4 days in RCCS and human hepatocytes, data were measured at 750 nm optical density. * p<0.05, HLSC 4d vs. non-differentiated HLSC; # p<0.05 HLSC 4d vs. HaCaT 4d.

### Experimental section

The experiments were carried out on Human Liver Stem Cells as described in WO2006126219. A schematic overview of the experiment is shown in Figure 1.

To characterize hepatic differentiation, the inventors analyzed the expression of stem markers and hepatic markers by qRT-PCR and immunofluorescence, and developed an updated Indocyanine green *in vitro* assay to measure hepatocyte functionality of the differentiated cells. The results obtained show that the assayed HLSCs are able to differentiate into hepatocyte-like cells characterized by significant upregulation in the expression of various markers such as hepatic genes, urea cycle enzymes and uptake transporters which are exclusively expressed on the sinusoidal side of mature hepatocytes after one day of differentiation. In addition, during differentiation, the cells were shown to downregulate stem cell markers such as KLF4 and others. Further analysis showed that the differentiated aggregates of hepatocyte-like cells release urea and FVIII into the supernatant as early as in the first 24 hours, which accumulate over time. In conclusion, the results obtained by the present inventors suggested that cultivation in a hepatic differentiation medium, preferably in a 3D rotary culture system, may promote rapid hepatic differentiation of HLSCs.

Use of a 3D rotary *in vitro* assay to generate functional hepatocyte-like cells from HLSCs is *per se* already known from the prior art (Herrera MB, Fonsato V, Bruno S, et al. Human liver stem cells improve liver injury in a model of fulminant liver failure. Hepatology. 2013;57(1):311-319. doi:10.1002/hep.25986). In the present description the inventors demonstrate that culturing of HLSCs in a hepatic differentiation medium in a 3D rotary culture system is able to generate functional and viable differentiated hepatocyte-like cells, which mature hepatic markers and stem cell markers expression profile is different from the HLSCs used as the starting material. However, the culture system is not a limiting feature of the method of the invention, as similar results may be obtained with other liquid culture systems.

### 2. Materials and Methods

### 2.1. Cell culture

Human liver stem cells (HLSC-ML11 Areta) derived from a GMP produced Master Cell Bank (MCB) were generated from 10-15 mm liver fragments obtained from liver donors, according to standard criteria of Centro Nazionale Trapianti, as described (Spada, M., Porta, F., Righi, D. et al. Intrahepatic Administration of Human Liver Stem Cells in Infants with Inherited Neonatal-Onset Hyperammonemia: A Phase I Study. Stem Cell Rev and Rep 16, 186-197 (2020). https://doi.org/10.1007/s12015-019-09925-z). Briefly, liver tissue was digested with GMP- grade collagenase (NBI, 0.6 mg/ml) and neutral protease (NBI, 0.73 mg/ml) (all from Nordmark Pharma GmbH, Germany) for 20 minutes at 37° C. The liver cell suspension was washed, centrifuged (400 g for 10 minutes) and cultured (4×10⁴ cell/cm²) in minimal essential medium (α-MEM) (Lonza, Basel, Switzerland) supplemented with 10% fetal calf serum (FCS, Euroclone, Milan, Italy), 4 ng/ml of human recombinant epidermal growth factor (EGF, Miltenyi Biotec, Bergisch Gladbach, Germany), 4 ng/ml of human recombinant fibroblast growth factor basic (FGF2, Miltenyi Biotec), 2 mM of L-glutamine (Lonza), and 100 U/ml of penicillin/streptomycin (from Sigma-Aldrich, St. Louis, MO, USA) and maintained in a humidified 5% CO₂ incubator at 37° C. HLSC, obtained after 2 weeks of culture, were seeded at the density of 3×10³ cells/cm² in T75 flasks in the same culture medium. Once the cells reached up to about 80% of confluency, they were harvested and centrifuged at 400 g for 5 minutes and used for experimental purposes. Cell identity was confirmed by FACS analysis by using anti CD44, CD105, CD73, CD90, CD29, CD14, CD34, CD45, and human Albumin. FITC, PE or APC conjugated antibodies were used.

HaCaT cells were kindly provided by Prof. Calautti (MBC, Turin). Cells were subcultured in Dulbecco Modified Eagle Medium (DMEM) high glucose (Thermo Fisher Scientific), supplemented with 2 mM L-glutamine and 10% FCS (Thermo Fisher Scientific). Cells were harvested using 1:1 mixture of EDTA (0.05%) and trypsin (0.1%) in 1X PBS without Ca²⁺ and Mg²⁺ to provide a physiologic osmolality.

HepG2 (purchase from ATCC, Virginia, USA) were cultured in DMEM supplemented with 10% FCS. Human hepatocytes (Hep) were purchase from Lonza.

### 2.2. In vitro hepatic 3D cell differentiation in rotary cell culture system (RCCS)

HLSC were differentiated into hepatocyte-like cells as previously described (Herrera MB, Fonsato V, Bruno S, et al. Human liver stem cells improve liver injury in a model of fulminant liver failure. Hepatology. 2013;57(1):311-319. doi:10.1002/hep.25986). Some modification of the original protocol was introduced, briefly, 5×10⁵ cells were resuspended in 10 mL of differentiation media composed as follows: 60/40 (v/v) mixture of low glucose DMEM (without phenol red, Euroclone) and MCDB-201 (without phenol red, Cell Culture Technologies, Lugano, Switzerland), supplemented with 2% FCS (Thermo Fisher Scientific), 0.026 g/ml ascorbic acid 3-phosphate, linoleic acid-bovine serum albumin (LA-BSA), insulin-transferrin-selenium (ITS) (all from Sigma), 10 ng/ml rhFGF4 and 10 ng/ml rhHGF (both from Miltenyi). Cells were inoculated in the rotary vessels (Synthecon, Houston, TX, USA) and rotated at 8 rpm/min (clockwise rotation). After 24 hours, 4, 7 and 10 days of culture, the vessels were emptied, cell aggregates were centrifuged at 300 g for 5 minutes, supernatants were collected, aliquoted and stored at -20°C. HLSC aggregates were washed in PBS 1X and used for the different experiments.

### 2.3. TUNEL assay

Apoptosis of differentiated HLSC aggregates was evaluated using the terminal dUTP nick end-labeling (TUNEL) assay (Merck Millipore, Darmstadt, Germany). 10 µm sections of OCT embedded aggregates were sliced, suspended in phosphate-buffered saline (PBS) and fixed in 1% paraformaldehyde in PBS, pH 7.4, for 15 minutes at 4 °C. This was followed by a secondary fixation with precooled ethanol/acetic acid (2:1) for 5 min at -20°C. Aggregates were treated with terminal deoxynucleotidyl transferase enzyme and incubated in a humidified chamber for 1 h at 37 °C and then treated with fluorescein isothiocyanate-conjugated anti-digoxigenin for 30 min at room temperature. After washing, samples were mounted in a medium containing 1 µg/ml of propidium iodide and the cells were analyzed by immunofluorescence microscopy.

### 2.4. PAS (Periodic Acid Schiff) Staining

Paraffin sections were deparaffinized and hydrated in water. The sections were incubated with 0.5% periodic acid solution for 5 minutes then stained with Schiff reagent for 15 minutes, followed by counterstaining with hematoxylin solution for 2 minutes. All steps were performed at room temperature, and sections were rinsed with tap water after each step.

### 2.5. Immunofluorescence staining

Immunofluorescence staining was performed as described previously (Herrera MB, Bruno S, Buttiglieri S, et al. Isolation and characterization of a stem cell population from adult human liver. Stem Cells. 2006;24(12):2840-2850. doi:10.1634/stemcells.2006-0114). HLSC were cultured on chamber slides (Nalgene Nunc International, Rochester, NY), fixed in 4% paraformaldehyde and permeabilized using Hepes-Triton X-100 buffer (Sigma-Aldrich). HLSC aggregates recovered at day 1, 4, 7 and 10 were directly embedded in OCT (Bio-Optica, Milan, Italy) and 10 µm slices were cut. Nonspecific antibody binding was blocked by incubation for 1 hour in PBS solution + 1% Bovine Serum albumin (BSA-Sigma) at room temperature.

The sections were stained with the following primary and secondary antibodies: anti-OCT3/4, anti-Nanog, anti-Sox2, anti-KLF4, anti-HNF4a, anti-human albumin, anti-OATP1B1, anti-cytokeratin 18, anti-cytokeratin 8, anti-aFP, anti-CYP1A1, anti-CYP3A4, anti-CYP7A1, anti-PCNA, anti-OTC, anti-ASS1, anti-CPS1, anti-ARG1, anti-ASL, anti-FVIII, anti-FIX and anti-FXI. Omission of the primary antibodies or substitution with nonimmune rabbit, rat, or mouse IgG was used as control. The secondary antibodies used were as follows; Alexa Fluor 488 goat anti-rabbit, mouse IgG and Alexa Fluor 546 goat antimouse IgG (both from Thermo Fisher Scientific, Waltham, MA, USA). Confocal microscopy analysis was performed using SP8 Confocal Microscope (Leica, Wetzlar, Germany). SlowFade Gold antifade reagent (Thermo Fisher Scientific, Eugene, OR, USA) was used for nuclear staining in combination with labeling & detection flour mount solution.

### 2.6. Western Blot

The cell aggregates were collected from RCCS and centrifuged at 400 g for 5 minutes at room temperature. The supernatants were aliquoted and cell aggregates were washed once in PBS 1X. After washing, PBS was removed and the aggregates were disrupted in liquid nitrogen and resuspended in a 50-70 µL RIPA buffer (RIPA + PMSF + protease inhibitors) (Sigma). The samples were kept on ice for 15 minutes to induce cell lysis and then centrifuged at 12,000 g for 15 minutes at 4°C. For the protein quantification, PierceTM BCA Protein Assay Kit (Thermo Fisher Scientific) was used according to the manufacturer's protocol and absorbance was read at 562 nm on a Promega microplate reader or iMark Microplate reader. Extracted proteins were then analyzed by SDS-PAGE and membranes were incubated with specific primary antibodies against urea cycle enzymes. GAPDH and Vinculin were used to normalize the data. Results were presented as Density unit and analyzed with ImagLab software (Bio Rad). For the analysis of release FVIII into the supernatant, the samples were concentrated ten times using 10kD Spin Columns (Abeam) following manufacturer's instructions.

### 2.7. Gene expression analysis by real time PCR

RNA of HLSC at p6/p7 and RCCS aggregates at day 1 and day 4 were isolated by miRNeasy Micro Kit (Qiagen, Hilden, Germany). RNA concentration was measured by Nanodrop ND-2000 (Thermo Fisher Scientific). The eluted RNA was stored at -80°C.

To perform the screening of liver related genes, a custom RT2 Profiler PCR Array was designed on GeneGlobe website (Qiagen) and a total of 1 µg total RNA was retro transcribed by RT2 First Strand Kit (Qiagen) according manufacturer's protocol. Cards were run on StepOnePlus real time PCR instruments (Applied Biosystem, Foster City, CA) using RT2 SYBR Green ROX^{™} qPCR Mastermix following the manufacturer's instructions. Five endogenous controls (ACTB, HPRT1, GUSB, GAPDH, B2M) were selected and retrotranscription, genomic contamination and reproducibility controls were added. Data were analyzed with online GeneGlobe software and results were provided as means of Fold Change (2-ΔΔCt) or with Expression Suite software (Applied Biosystem). ΔΔCt is the normalized gene expression (2-ΔCt) in the Test Sample divided into the normalized gene expression (2-ΔCt) in the Control Sample. Biological replicates of each condition were run in duplicate. The complete list of analyzed genes is provided in Table 1 below:

**Table 1**

| **Refseq** | **Symbol** | **Description** | **Name** |
|---|---|---|---|
| NM_0004 63 | UGT1A1 | UDP glucuronosyltransferase 1 family, polypeptide A1 | BILIQTL1/GNT1/HUG-BR1/UDPGT/UDPGT 1-1/UGT1/UGT1A |
| NM_0190 93 | UGT1A3 | UDP glucuronosyltransferase 1 family, polypeptide A3 | UDPGT/UDPGT 1-3/UGT-1C/UGT1-03/UGT1.3/UGT1A3S/UGT1C |
| NM 0071 20 | UGT1A4 | UDP glucuronosyltransferase 1 family, polypeptide A4 | HUG-BR2/UDPGT/UDPGT 1-4/UGT-1D/UGT1-04/UGT1.4/UGT1A4S/UGT1D |
| NM_0027 01 | POUF5F 1 | POU class 5 homeobox 1 | OCT3/OCT4/OTF-3/OTF3/OTF4/Oct-3/Oct-4 |
| NM 0224 54 | SOX17 | SRY (sex determining region Y)-box 17 | VUR3 |
| NM_0217 84 | FOXA2 | Forkhead box A2 | HNF3B/TCF3B |
| NM 0022 73 | KRT8 | Keratin 8 | CARD2/CK-8/CK8/CYK8/K2C8/K8/KO |
| NM_0002 24 | KRT18 | Keratin 18 | CK-18/CYK18/K18 |
| NM 0022 76 | KRT19 | Keratin 19 | CK19/K19/K1CS |
| NM_0043 64 | CEBPA | CCAAT/enhancer binding protein (C/EBP), alpha | C/EBP-alpha/CEBP |
| NM_0051 94 | CEBPB | CCAAT/enhancer binding protein (C/EBP), beta | C/EBP-beta/IL6DBP/NF-IL6/TCF5 |
| NM_0005 45 | HNF1A | HNF1 homeobox A | HNF-1A/HNF1/IDDM20/LFB1/MODY3/T CF-1/TCF1 |
| NM_0004 58 | HNF1B | HNF1 homeobox B | FJHN/HNF-1 -beta4fNF-1 B/HNF 1 beta/HNF2/HP C 1 1/LF-B3/LFB3/MODY5/TCF-2/TCF2/VHNF1 |
| NM_1788 49 | HNF4A | Hepatocyte nuclear factor 4, alpha | FRTS4/HNF4/HNF4a7/HNF4a8/HNF 4a9/HNF4alpha/MODY/MODY1/NR 2A1/NR2A21/TCF/TCF14 |
| NM_0044 98 | ONECU T | One cut homeobox 1 | HNF-6/HNF6/HNF6A |
| NM_0002 95 | SERPIN A1 | Serpin peptidase inhibitor, clade A (alpha-1 antiproteinase, antitrypsin), member 1 | A1A/A1AT/AAT/PI/PI1/PRO2275/alp halAT |
| NM 0004 77 | ALB | Albumin | ANALBA/FDAH/PR00883/PR00903 /PRO1341 |
| NM_0011 34 | AFP | Alpha-fetoprotein | AFPD/FETA/HPAFP |
| NM_0002 77 | PAH | Phenylalanine hydroxylase | PH/PKU/PKU1 |
| NM_0004 99 | CYP1A1 | Cytochrome P450, family 1, subfamily A, polypeptide 1 | AHH/AHRR/CP11/CYP1/P1-450/P450-C/P450DX |
| NM_0007 61 | CYP1A2 | Cytochrome P450, family 1, subfamily A, polypeptide 2 | CP12/P3-450/P450(PA) |
| NM_0001 04 | CYP1B1 | Cytochrome P450, family 1, subfamily B, polypeptide 1 | CP 1B/CYPIB1 /GLC 3 A/P45 01B1 |
| NM_0007 67 | CYP2B6 | Cytochrome P450, family 2, subfamily B, polypeptide 6 | CPB6/CYP2B/CYP2B7/CYP2B7P/C YPIIB6/EFVM/IIB 1/P450 |
| NM_0007 71 | CYP2C9 | Cytochrome P450, family 2, subfamily C, polypeptide 9 | CPC9/CYP2C/CYP2C10/CYPIIC9/P4 50IIC9 |
| NM_0174 60 | CYP3A4 | Cytochrome P450, family 3, subfamily A, polypeptide 4 | CP33/CP34/CYP3A/CYP3A3/CYPIII A3/CYPIIIA4/HLP/NF-25/P450C3/P450PCN1 |
| NM_0007 65 | CYP3A7 | Cytochrome P450, family 3, subfamily A, polypeptide 7 | CP37/CYPIIIA7/P-450(HFL33)/P-450111A7/P450-HFLA |
| NM_0007 80 | CYP7A1 | Cytochrome P450, family 7, subfamily A, polypeptide 1 | CP7A/CYP7/CYPVII |
| NM_0018 75 | CPS1 | Carbamoyl-phosphate synthetase 1, mitochondrial | CPSASE1/PHN |
| NM_0000 50 | ASS1 | Argininosuccinate synthase 1 | ASS/CTLN1 |
| NM_0005 31 | OTC | Omithine carbamoyltransferase | OCTD |
| NM_0000 45 | ARG1 | Arginase, liver | - |
| NM_0000 48 | ASL | Argininosuccinate lyase | ASAL |
| NM_0011 01 | ACTB | Actin, beta | BRWS1/PS1TP5BP1 |
| NM_0040 48 | B2M | Beta-2-microglobulin | - |
| NM_0001 94 | HPRT1 | Hypoxanthine phosphoribosyltransferase 1 | HGPRT/HPRT |
| NM_0020 46 | GAPDH | Glyceraldehyde-3-phosphate dehydrogenase | G3PD/GAPD/HEL-S -162eP |
| NM_0001 81 | GUSB | Glucuronidase, beta | BG/MPS7 |
| SA_00104 | RTC | Reverse Transcription Control | RTC |
| | PPC | Inter Plate Reproducibility Control | PPC |
| SA_00105 | HGDC | Human Genomic DNA Contamination | HIGX1A |

For specific gene expression analysis, quantitative real-time PCR was performed. Briefly, first-strand cDNA was synthesized from 200 ng of total RNA using the High-Capacity cDNA Reverse Transcription Kit (Applied Biosystems). qRT-PCR was performed using the StepOnePlus machine (Applied Biosystems,) in a 20 µl reaction mixture containing 5 ng or 10 ng of cDNA template (according to tested gene expression analysis), the sequence-specific oligonucleotide primers (purchased from MWG Biotech, Eurofins Scientific, Brussels, Belgium), and the Power SYBR Green PCR Master Mix (Applied Biosystems). TBP was used as endogenous control. Fold change expressions with respect to control were calculated for all samples using the ΔΔCt method. The primers used for qRT-PCR are reported in Table 2 below:

**Table 2**

| Gene name | Forward primer (5'-3') | SE Q ID NO | Reverse primer (5'-3') | SE Q ID NO |
|---|---|---|---|---|
| h-SLCO1 B1 | ACTGATTCTCGATGGGTT GGAG | 1 | TGTTTCCAGCACATGCAAA GAC | 2 |
| h-SLAC10 A | TGTTTCCAGCACATGCAA AGAC | 3 | ATCAAGAGTGGTGTCATGG CAA | 4 |
| h-NANO G | ACAACTGGCCGAAGAATA GCA | 5 | GGTTCCCAGTCGGGTTCA | 6 |
| h-OCT4 | AGCAGGAGTCGGGGTGG | 7 | AGAGCTTTGATGTCCTGGG ACT | 8 |
| h-SOX2 | TGCGAGCGCTGCACAT | 9 | GCAGCGTGTACTTATCCTTC TTCA | 10 |
| h-KLF4 | CCATAACCAAGAGCTCAT GCC | 11 | GGGCCACGATCGTCTTCC | 12 |
| h-cMYC | CAGCGACTCTGAGGAGGA ACA | 13 | TGAGGAGGTTTGCTGTGGC | 14 |
| h-TBP | TGTGCACAGGAGCCAAGA GT | 15 | ATTTTCTTGCTGCCAGTCTG G | 16 |
| h-FV III | CTGTGGACGCAAGATTTC CTC | 17 | AGGATGGGAAGCCATGTTC TT | 18 |
| h-FIX | AGTCCTGTGAACCAGCAG TGC | 19 | GGGTGCTTTGAGTGATGTTA TCC | 20 |
| h-FXI | CATCCCAGTGTTCTGCCAT TC | 21 | AGGTAAAAAACTGGCAGCG GA | 22 |

### 2.8. Urea quantification by HPLC-MS analysis

Urea production was measured by liquid chromatography hyphenated with tandem mass spectrometry. Urea chromatographic separations were run on a Nexera HPLC (Shimadzu, Kyoto, Japan) coupled to a Qtrap mass spectrometer Sciex 5500 (Sciex, Framingham, MA, USA), equipped with an ESI Turbo Ion Spraysource. Supernatant samples from RCCS experiments, were analyzed using a hydrophilic interaction liquid chromatography (HILIC) column (Phenomenex Luna HILIC 150 × 2.1 mm, 3 µm particle size, Phenomenex, Torrance, CA, USA) at a flow rate of 200 µL/min. A gradient mobile phase composition was adopted: 95/5 to 20/80 solvent A/solvent B. Solvent A was acetonitrile and solvent B 5 mM aqueous ammonium acetate with 2% of acetonitrile. The injection volume was 20 µL. The tuning parameters adopted for the ESI source were source voltage 4.5 kV (positive ion mode), source temperature 300°C, curtain gas nitrogen. Analyses were run using selected reaction monitoring MS/MS acquisition, precursor ion 61 m/z, product ion 44 m/z, collision energy 20 V. Lower limit of quantification (LLOQ) was 50 ng/mL (supernatant samples were tenfold diluted with acetonitrile before injection). Data were expressed as ppm (µg/mL). Urea external standard (Sigma-Aldrich) curve was used for quantifying the concentration of the samples.

### 2.9. Hepatocyte functional assay: Indocyanine green (ICG) uptake

Indocyanine Green (ICG) (ICG, USP, Frederick, MD, USA) was dissolved in DMSO at 33 mg/mL, then added to alpha MEM (without Phenol red, Lonza, Basel) to a final concentration of 0.5 mg/mL. After incubation at 37 °C for 60 min, the medium with ICG was blocked using FCS, discarded and the cells or aggregates were washed three time with PBS. The cellular uptake of ICG was then examined by microscopy and some representative pictures were taken using Moticam BTX8Camera (Motic, Xiamen, China) . ICG uptake quantification was performed after lysis of the aggregates or the cells using 400 µL of RIPA buffer. ICG concentration was quantified by absorbance measurement at 750 nm with a plate reader (iMark Microplate reader, Bio-Rad) and plotted against a standard curve of ICG. Measurements were performed in duplicates. HaCaT and non-differentiated HLSC were used as negative control, while hepatocytes (Lonza) were used as positive control.

### 2.10. Statistical Analysis.

Raw data were analyzed by GraphPad Prism 6.0 software (GraphPad Software, San Diego, CA, USA). Results are expressed as a mean value of 3-5 independent biological replicates. Statistical analyses were performed by employing a one-way analysis of variance (ANOVA) followed by Newman-Keuls test or Student's t-test. A p value of <0.05 was considered significant.

### 3. Results

### 3.1 HLSC characterization

HLSC were isolated from a human liver biopsy in GMP conditions as described previously (Spada M, Porta F, Righi D, et al. Intrahepatic Administration of Human Liver Stem Cells in Infants with Inherited Neonatal-Onset Hyperammonemia: A Phase I Study. Stem Cell Rev Rep. 2020;16(1):186-197. doi:10.1007/s12015-019-09925-z). Before the experiments, a Working Cell Bank (WCB) at passage 6 was created and cells were kept frozen ready to use at the moment of the experiments. During the expansion, HLSC-WCB maintained a spindle-shaped morphology, which was observed until the cell reached over 80 percent confluency as shown in Figure 2a. The proliferative potential and cell bank reproducibility was evaluated by measuring the cumulative population doubling time (CPDT) of the cell after expansion in 2D. As shown in Figure 2b, there were no statistical differences in the CPDT up to passage 6.

To confirm the identity of the cells, a panel of surface markers were analyzed by fluorescence-activated cell sorter (FACS) analysis. The analysis showed that HLSCs had a high expression (≥90%) of CD29, CD73, CD90, CD105 and CD44 and were negative for the immune cell marker CD14 and hematopoietic cell marker CD45. In addition, the expression of albumin was also confirmed (Figure 2c). The western blot analysis revealed that HLSCs were positive for some stem cell markers such as OCT3/4, Nanog, Sox2 and KLF4 and the mature hepatic marker albumin (Figure 2c).

### 3.2 In vitro Hepatic differentiation of HLSC cultured in Rotary Cell Culture System (RCCS)

A schematic overview of the performed experiment is shown in Figure 3a. The cells were maintained in differentiation conditions from day 1 up to day 10. HLSC aggregates were recovered and analyzed by PCNA, TUNEL and PAS staining after 1, 4, 7 and 10 days of culture (Figure 3a). PCNA staining showed that during differentiation HLSC did not proliferate (Figure 3b). Apoptosis was detected in hepatocyte-like differentiated HLSC aggregates as confirmed by TUNEL assay (Figure 3c). The inventors observed that at 4 days 11.9±3.2% of dead cells were present in the HLSC aggregates. This percentage increased rapidly over time reaching 77.1±6.1% at 10 days (Figure 3d). The apoptotic events seen could be due to the exhaustion of nutrients in the media as well as the absence of external oxygen inside the culture rotary vessels as the number of days of differentiation increases.

Periodic acid-Schiff (PAS) staining is used to identify glycogen synthesis and hepatocyte function; As shown in Figure 3e, the inventors observed the presence of some PAS positive area on the surface of the aggregates suggesting that the glycogen active producing cells are present on the outer part of the structure. Based on TUNEL and PAS staining results, the inventors observed that the performance of the assay is optimal until day 4, although some experiments were performed also at day 7. In addition, the inventors showed a significantly reduction of total amount of RNA from RCCS HLSC after 7 days, confirming the loss of cells at this timepoint.

### 3.3 Expression of stem cell markers after hepatocyte-like differentiation

To evaluate the maturation state of HLSC, the inventors compared the expression of pluripotency associated genes among non-differentiated HLSC culture in plastic adhesion and HLSC aggregates cultured for 1 and 4 days in RCCS (Figure 4). By immunofluorescence analysis the inventors showed that HLSCs expressed Nanog, SOX2, KLF4 and OCT3/4 (Figure 4a). The gene expression of all the stem cell markers was compared to non-differentiated HLSC (Figure 4b). As shown in Figure 4b, Nanog, SOX2 and OCT3/4 expression peaked at day 4 in RCCS 3D cultures and were subsequently downregulated as the days of differentiation progressed (data not shown). These markers were shown to be correlated to self-renewal, pluripotency, and reprogramming capacity (REF). The pattern of expression of Nanog and OCT 3/4 changed from non-differentiated HLSC to differentiated, with the later showing a cytoplasmic distribution rather than nuclei. In contrast, the expression of KLF4 was decreased over time in RCCS (Figure 4a) which also correlated at the molecular gene level (Figure 4b).

### 3.4 Expression of Hepatic markers in hepatocyte-like differentiated HLSC.

HLSC aggregates were characterized to evaluate the expression of specific proteins expressed by the liver, suggesting the efficiency of our liver differentiation system. As previously shown (REF), albumin is well expressed by HLSC (Figure 5a). Albumin pattern was distributed differently in RCCS with respect to non-differentiated HLSC, and the expression of albumin mRNA increased during differentiation in RCCS (Figure 5b). HNF4 is well expressed by non-differentiated HLSCs and its expression is maintained during differentiation. Instead CK8, is expressed at very low levels in non-differentiated HLSCs and increased over the course of differentiation in the RCCS (Figure 5a and b) both at protein and gene levels. On the other hand, Alpha Fetoprotein (αFP), a protein identified as an immature marker of hepatoblast which has been previously demonstrated to be positive in non-differentiated HLSCs, reduced significantly in the cells post differentiation in the RCCS both at a protein and gene level (Figure 5a, b).

Cytochrome P450 enzymes are markers of mature functional hepatocytes. Specific P450 Cytochrome isoforms CYP1a1, CYP3a4, and CYP7a1 are expressed at low levels in HLSC and increase during differentiation (Figure 6a, b). At day 4, the expression significantly increased (Figure 6a) compared to undifferentiated HLSCs. In addition, the PCR array showed an increase in the gene expression of CYP1b1, CYP3a7 after 1 day and that persisted after 4 days of differentiation, while CYP1a2 and CYP2b6 showed a higher expression at day 4 (Figure 7 and Table 3 below).

**Table 3**

| Sample | Nucleic Acid (ng/µl) |
|---|---|
| HLSC sample 1 | 452.4 |
| HLSC sample 2 | 478.7 |
| HLSC A sample 3 | 471.6 |
| RCCS 1d sample 1 | 636.1 |
| RCCS 1d sample 2 | 672.2 |
| RCCS 1d sample 3 | 586.2 |
| RCCS 4d sample 1 | 536.6 |
| RCCS 4d sample 2 | 387.7 |
| RCCS 4d sample 3 | 134.8 |
| RCCS 7d sample 1 | 70.0 |
| RCCS 7d sample 2 | 75.5 |
| RCCS 7d sample 3 | 70.9 |

The gene expression profiles of non-differentiated HLSC vs. HLSC differentiated in RCCS were compared by PCR array (Figure 7). As shown in the heatmap (Figure 7), clustering analyses revealed significant changes in the expression of hepatic genes in all the groups. Ten genes out of 32 key genes were upregulated and 4 were downregulated in HLSC cultured in RCCS after day 1, and 19 genes out of 32 key genes were upregulated and 3 were downregulated in HLSC cultured in RCCS after 4 days (Figure 7). The majority of these genes encoded for hepatic signaling molecules involved in the development of liver. The list of upregulated genes included CK8 (krt8), CK18 (krt18), UGT1A3, UGT1A4, HNF1A, PAH, CYP1A1, CYP1A2, CYP1B1, CYP2B6, CYP3A4, CYP3A7, CYP7A1, CPS1, ASS1, OTC, ARG1, and ASL.

### Expression of coagulation factors in in hepatocyte-like differentiated HLSC

Following 3D differentiation of HLSC, gene expression of coagulation factors involved in the common coagulation pathways, and in the thrombolysis and hemostasis processes were present in non-differentiated HLSC (Figure 8 and Figure 9). Notably, after 4 days in RCCS, coagulation factor VIII (FVIII) (Figure 8b) was downregulated in differentiated HLSC both at the protein and gene levels (Figure 8a, b). In addition, during 3D culture conditions, cells secreted FVIII into the supernatant with a peak release at 4 days (Figure 8c). In contrast, FIX and FXI (Figure 9) gene expression were significantly higher in RCCS HLSC when compared with non-differentiated HLSC (Figure 9).

### Urea secretion quantitation and expression of urea cycle enzymes

Urea cycle is the predominant mechanism for the clearance of waste nitrogen resulting from protein turnover in the liver. This energy-dependent process occurs first in mitochondria and ends in the cytoplasm. When this process is not working efficiently, toxic ammonia (NH₃) accumulates within the body and may elicit clinical manifestations such as lethargy, slurred speech, cerebral edema, and asterixis.

Urea quantitation was proposed as a potency assay to evaluate HLSC differentiation and regeneration capacity. Urea was measured by mass spectrometry in RCCS supernatants compared to medium alone after 1, 4 and 7 days. Urea is secreted after day 1 and its level remains unchanged until day 7, suggesting that most of the production and release happens very early during the induction of differentiation (Figure 10).

The inventors evaluated the expression of urea cycle enzymes in basal (undifferentiated) and differentiated HLSC (Figure 11). HLSCs at a basal level expressed low levels of Carbamoyl-Phosphate Synthetase 1 (CPS1), Arginino-succinate lyase (ASL) and Arginase 1 (ARG1), high levels of Arginino-succinate Synthase 1 (ASS1) and do not express Ornithine Transcarbamylase (OTC) (Figure 11a). After 1 day of differentiation both protein and gene levels of the 5 enzymes increased, reaching a peak of expression, with the exception of the molecular level of ASS1 (Figure 11a, b). Ornithine Transcarbamylase (OTC) is not expressed by HLSC, but its expression slightly increases during differentiation (Figure 11a- b).

The Western blot analysis of urea cycle enzymes revealed an increase at the protein level but no significant differences between the non-differentiated HLSC and HLSC aggregates in RCCS was observed (Figure 12 a,b). However, three-dimensional conditions significantly increased the level of CPS1 proteins at both 4 and 7 days of culture in RCCS (Figure 12 a,b).

### 3.5 Indocyanine green uptake by hepatocyte-like HLSC differentiated in RCCS

Indocyanine green is an anionic dye that is used to evaluate liver functionality. The ICG uptake is due to a membrane transporter of the OATP family, the isoform OATP1B1 typically associated with mature hepatocytes.

To evaluate if the uptake was mediated by the presence of the specific hepatic transporters, the inventors performed immunofluorescence and real time analyses to evaluate the expression of OATP1B1. The expression of the transporter was visibly increased already at 4 day (Figure 13 b-c) and decreased after 7 days, as seen by PCR analysis (Figure 13c).

As shown in Figure 13d, ICG uptake is evident in HLSC after RCCS, but not in HaCaT cells (Human keratinocytes) neither non-differentiated HLSC. Furthermore, the uptake of ICG from HLSC in RCCS was significantly higher than from the HaCaT negative control (Figure 13b-c) after 4 days, demonstrating that HLSC are able to differentiate into functional hepatocytes.

Current hepatic differentiation protocols that give rise to hepatic-like cells from MSC and iPSCs are highly heterogeneous and limited by the low differentiation efficiency due to differences from cell sources that contribute to varying levels of expansion under different culture conditions.

The results obtained by the present inventors show that hepatic differentiation of HLSCs in a 3D rotary device led to a consistently better performance compared to previous works insofar as a wide spectrum of *in vitro* hepatic functions is concerned. Such hepatic functions include, *inter alia,* liver-specific gene expression, urea cycle enzymes, cytochrome P450, transmembrane transporters, and a wide range of coagulation factors at both gene and protein level. In addition, urea, albumin and FVIII secretion was improved.

## Claims

1. A method of qualifying as pharmaceutically active a batch of undifferentiated adult stem cells having the potential of differentiating along the hepatic lineage, the method comprising the following steps:
i) obtaining a cell sample from said batch and culturing the cells in a hepatic differentiation medium, whereby hepatocyte-like cells are obtained;
ii) performing a functional assay on said hepatocyte-like cells, wherein the functional assay is one or a combination of the following assays:
- detecting Indocyanine green (ICG) uptake,
- detecting the expression of the transporter OATP1B1,
- detecting the expression of one or more markers selected from the group consisting of Nanog, SOX2, KLF4, OCT3/4, CK8, αFP, Cyp1A1, CYP3A4, Cyp7A1, FVIII, FIX, FXI, ASL and ARG;
iii) performing the same functional assay as in step ii) on a negative control cell sample; and
iv) comparing the results of the functional assay of step ii) with the results of the functional assay of step iii),
wherein said batch is qualified as pharmaceutically active if the results of the functional assay of step ii) are statistically significantly different from the results of the functional assay of step iii), wherein statistical significance is determined based on a p-value ≤ 0.05.

2. The method according to claim 1, wherein the functional assay of step ii) is performed after a period of 1 to 10 days, preferably 1 to 7 days, more preferably 4 to 7 days of culturing in the hepatic differentiation medium.

3. The method according to claim 1 or 2, wherein said undifferentiated adult stem cells having the potential of differentiating along the hepatic lineage are human liver pluripotent progenitor cells or bone marrow mesenchymal stem cells.

4. The method according to claim 3, wherein said are human liver pluripotent progenitor cells are non-oval human liver stem cells.

5. The method according to any one of claims 1 to 4, wherein the negative control cell sample is a sample of the undifferentiated adult stem cells having the potential of differentiating along the hepatic lineage which are not cultured in a hepatic differentiation medium.

6. The method according to any one of claims 1 to 5, wherein the negative control cell sample is a sample of a cell line not having the potential of differentiating along the hepatic lineage.

7. The method according to claim 6, wherein the negative control cell sample is a sample of the HaCat keratinocytes.

8. The method according to any one of claims 1 to 7, wherein the functional assay further includes detecting urea secretion.

9. The method according to any one of claims 1 to 8, wherein in step i) the hepatic differentiation medium includes hepatocyte growth factor and/or fibroblast growth factor 4.

10. The method according to any one of claims 1 to 8, wherein in step i) the hepatic differentiation medium includes epidermal growth factor, insulin and dexamethasone.

11. The method according to any one of claims 1 to 10, wherein in step i) the cells are cultured in a 3D rotary device.

12. The method according to any one of claims 1 to 11, wherein qualifying as a pharmaceutically active substance is or comprises qualifying as suitable for establishing a Master Cell Bank.

13. The method according to any one of claims 1 to 11, wherein qualifying as a pharmaceutically active substance is or comprises qualifying as suitable for treating a health condition in a human subject in need thereof.

14. The method according to claim 13, wherein the health condition is selected from the group consisting of fibrotic, metabolic, and oncologic diseases.

15. The method according to claim 13, wherein the health condition is selected from the group consisting of fibrotic, metabolic, and oncologic diseases or disorders involving the liver, the biliary tree, the kidney, the heart muscle, the lungs or the pancreas.

16. The method according to claim 13, wherein the health condition is selected from the group consisting of urea cycle disorders, Crigler-Najiar, alpha1antitrypsin deficiency, biliary atresia, acute liver failure, nonalcoholic fatty liver disease, nonalcoholic steatohepatitis, primary biliary cholangitis, autoimmune hepatitis, cirrhosis, hepatocellular carcinoma, cholangiocarcinoma, liver genetic metabolic disorders, primary sclerosing cholangitis, chronic kidney disease, focal segmental sclerosis, diabetic nephropathy, membranous glomerulonephritis, diabetes type I and II, cardiac fibrosis, idiopathic pulmonary fibrosis, metastatic hepatic tumors, renal cell carcinoma, and lung carcinoma.

17. Use of a batch of undifferentiated adult stem cells having the potential to differentiate along the hepatic lineage and which has been qualified as pharmaceutically active according to the method of any one of claims 1 to 16, for establishing a Master Cell Bank.
